(19) Europäisches Patentamt
European Patent Office
Office européen des brevets

(11) Veröffentlichungsnummer : **0 082 814**
**B1**

# (12) EUROPÄISCHE PATENTSCHRIFT

(45) Veröffentlichungstag der Patentschrift : **04.03.87**

(51) Int. Cl.⁴ : **C 12 N 1/20**, C 12 N 1/32, C 02 F 3/34// C12R1/38

(21) Anmeldenummer : **82810527.0**

(22) Anmeldetag : **06.12.82**

## (54) Mikroorganismen des Genus Pseudomonas und Verfahren zum Abbau von Methylgruppen-haltigen Verbindungen in wässrigen Lösungen.

(30) Priorität : **11.12.81 CH 7933/81**

(43) Veröffentlichungstag der Anmeldung : **29.06.83 Patentblatt 83/26**

(45) Bekanntmachung des Hinweises auf die Patenterteilung : **04.03.87 Patentblatt 87/10**

(84) Benannte Vertragsstaaten : **AT BE CH DE FR GB IT LI LU NL SE**

(56) Entgegenhaltungen :
**CHEMICAL ABSTRACTS, Band 92, Nr. 3, 21. Januar 1980, Seite 527, Spalte 1, Nr. 20540d, Columbus, Ohio, USA. T.A. RAYBUSHKO et al., "Growth and biomass accumulation of Pseudomonas strains in nutrient media with ethanol"**
**CHEMICAL ABSTRACTS, Band 88, Nr. 19, 8. Mai 1978, Seite 391, Spalte 1, Nr. 134840z, Columbus, Ohio, USA. N.M. GORNAK et al., "Representatives of the Pseudomonas genus, potential producers of food protein from ethanol"**
**JOURNAL OF BACTERIOLOGY, Band 122, Nr. 3, Juni 1975, Seiten 905-910, Baltimore, Md (US). C. WAGNER et al., "Enzymes involved in the assimilation of one-cabon units by Pseudomonas MS"**
**AGRICULTURAL AND BIOLOGICAL CHEMISTRY, Band 40, Nr. 11, November 1976, Seiten 2129-2135, Tokyo (JP).J.S. ROCK et al., "Isolation and characterization of two methanol - utilizing bacteria"**
**INTERNATIONAL BIODETERIOR BULLETIN, Band 14, Nr. 3, 1978, Seiten 77-83, BIRMINGHAM (GB). J.A. MACKRELL et al., "The biodegradation of quarternary ammonium compounds"**
**CHEMICAL ABSTRACTS, Band 81, Nr. 16, 21. Oktober**

(73) Patentinhaber : **CIBA-GEIGY AG**
**Klybeckstrasse 141**
**CH-4002 Basel (CH)**

(72) Erfinder : **Ghisalba, Oreste, Dr.**
**Feldbergstrasse 91**
**CH-4057 Basel (CH)**
Erfinder : **Heinzer, Franz, Dr.**
**chemin des minoux 17**
**CH-2900 Porrentruy (CH)**
Erfinder : **Küenzi, Martin, Dr.**
**Hüslimattstrasse 16**
**CH-4132 Muttenz (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

1974, Seite 299, Spalte 2, Nr. 96040j, Columbus, Ohio, USA. A. GRABINSKA-LONIEWSKA, "Activated sludgebacteria participating in the biodegradation of methanol, formaldehyde, and ethylene glycol.I.isolation and indentification"
BIOCHEMICAL SOCIETY TRANSACTIONS, Band 1, 1973, Seiten 667-668, London (GB). D. HAMPTON et al.: "The metabolism of tetramethylammonium chloride by bacterium 5H2"
BIOCHEMICAL JOURNAL, Band 148, Nr. 3, Seiten 505-511, London (GB). J. COLBY et al.: "Tricarboxylic acid-cycle and related enzymes in restricted facultative methylotrophs"

**Beschreibung**

Die vorliegende Erfindung betrifft neue fakultativ methylotrophe Mikroorganismen des Genus Pseudomonas, ein Verfahren zur mikrobiologischen Reinigung von wässrigen Lösungen durch Abbau von Trimethyläthylammoniumsalze in Gegenwart dieser Mikroorganismen, die Zellmasse, welche von diesen Mikroorganismen hergestellt wird, sowie die Verwendung der verfahrensgemäss erhältlichen Zellmasse.

Unter dem Begriff « methylotrophe Mikroorganismen » versteht man solche Mikroorganismen, die auf Nährmedien wachsen, welche als Kohlenstoffquelle Verbindungen mit nur einem Kohlenstoffatom, z. B. Methanol, enthalten.

Unter dem Begriff « fakultativ methylotrophe Mikroorganismen » versteht man solche Mikroorganismen, die auf Nährmedien wachsen, welche als Kohlenstoffquelle Verbindungen mit einem Kohlenstoffatom, z. B. Methanol, und/oder Verbindungen mit mehreren Kohlenstoffatomen, z. B. Glucose, enthalten.

In der Literatur sind fakultativ methylotrophe Mikroorganismen beschrieben, welche in wässriger Lösung eine oder mehrere Verbindungen aus der folgenden Gruppe von organischen Verbindungen abbauen bzw. als Kohlenstoff- oder als Kohlenstoff- und Stickstoffquelle verwerten können : Methanol, Aethanol, Acetate, z. B. Natriumacetat, Monosaccharide, z. B. Glucose, Disaccharide, z. B. Saccharose, bestimmte Methylammoniumverbindungen, z. B. Trimethylammonium-, Aethylmethylammonium- oder Aethyldimethylammoniumchlorid oder die freien Amine von diesen Salzen oder Trimethylaminoxid.

Solche Mikroorganismen sind in verschiedenen Stammsammlungen, z. B. in der American Type Culture Collection (ATCC), in der Deutschen Sammlung von Mikroorganismen (DSM) oder in der National Collection of Industrial Bacteria (NCIB), hinterlegt und in den von diesen Hinterlegungsstellen publizierten Katalogen aufgeführt.

Einige fakultativ methylotrophe Mikroorganismen, die in wässriger Lösung in Gegenwart von Tetramethylammoniumchlorid wachsen, sind von J. Colby und von L.J. Zatmann, Biochem. J., *148,* 505-511 (1975) und von D. Hampton und L.J. Zatmann in Biochem. Soc. Trans. *1,* 667-668 (1973) ohne Angabe einer Hinterlegungsstelle beschrieben. Sie sind ausserdem in keinen von Hinterlegungsstellen publizierten Katalogen aufgeführt.

Mackrell, Y.A. and Walker, J.R.L. beschreiben in Int. Biodeterior. Bull. 14 (3), 1978 (77-83) die prinzipielle Abbaubarkeit von quaternären Ammoniumverbindungen, z. B. Trimethyläthylammoniumchlorid in wässriger Lösung durch Mikroorganismen. In dieser Publikation fehlt eine Charakterisierung der betreffenden Mikroorganismen, eine Herkunftsangabe und die Angabe der Isolierungsmethode und der Hinterlegungsstelle.

Bei der grosstechnischen Produktion in der chemischen Industrie fallen wässrige Lösungen, z. B. Abwässer, an, welche Trimethyläthylammoniumsalze zum Teil in sehr hohen Konzentrationen als Verunreinigungen enthalten. Um Belastungen für die Umwelt durch diese Verbindungen zu vermeiden, muss man solche Abwässer reinigen.

Dieses Problem wird durch die vorliegende Erfindung gelöst, welche neue fakultativ methylotrophe Mikroorganismen des Genus Pseudomonas betrifft, welche Trimethyläthylammoniumsalze abbauen können. Die vorliegende Erfindung betrifft ein Verfahren zur mikrobiologischen Reinigung von wässrigen Lösungen durch Abbau von Trimethyläthylammoniumsalzen in Gegenwart dieser Mikroorganismen, die Zellmasse, welche von diesem Mikroorganismen hergestellt wird, sowie die Verwendung der verfahrensgemäss erhältlichen Zellmasse.

Die vorliegende Erfindung betrifft Mikroorganismen des Genus Pseudomonas aus der Gruppe folgender Stämme : Pseudomonas TMEA 14 (NRRL-B-12582), TMEA 81 (NRRL-B-12581), TMEA 83 (NRRL-B-12580), TMEA 84 (NRRL-B-12579), TMEA 86 (NRRL-B-12578), TMEA 87 (NRRL-B-12577), TMEA 89 (NRRL-N-12576), TMEA 199 (NRRL-B-12575) und TMEA (NRRL-B-12574) und die davon abgeleiteten Mutante und ein Verfahren zur mikrobiologischen Reinigung von wässrigen Lösungen durch Abbau von Trimethyläthylammoniumsalzen.

Die neuen Mikroorganismen stammen aus dem Klärschlamm einer Abwasserreinigungsanlage der Ciba-Geigy AG und sind bei der Agricultural Research Culture Collection (NRRL) in Peoria, Illinois 61604, USA, am 3. November 1981 hinterlegt worden. In der folgenden Tabelle sind Hinterlegungs-Nr. und Isolierungsmethode für jeden einzelnen Stamm angegeben.

Tabelle 1

| Bezeichnung | | Hinterlegungs- | Isolierungs- |
|---|---|---|---|
| Genus | Kenn. Nr. | Nr. (NRRL) | methode |
| Pseudomonas | TMEA 14 | B-12582 | 1 |
| Pseudomonas | TMEA 81 | B-12581 | 1 |

Tabelle 1 (Suite)

| Bezeichnung | | Hinterlegungs- Nr. (NRRL) | Isolierungs- methode |
|---|---|---|---|
| Genus | Kenn. Nr. | | |
| Pseudomonas | TMEA 83 | B-12580 | 1 |
| Pseudomonas | TMEA 84 | B-12579 | 1 |
| Pseudomonas | TMEA 86 | B-12578 | 1 |
| Pseudomonas | TMEA 87 | B-12577 | 1 |
| Pseudomonas | TMEA 89 | B-12576 | 1 |
| Pseudomonas | TMEA 199 | B-12575 | 2 |
| Pseudomonas | TMEA 211 | B-12574 | 2 |

Isolierungsmethode 1

Eine Abwasserprobe oder eine Klärschlammsuspension (1 g pro 10 ml sterilem Wasser) wird auf sterilem Trimethyläthylammoniumchlorid-haltigem Agar (auf der Basis von Nährlösung MV 7) plattiert. Die Nährlösung MV 7 enthält in einem Liter Wasser die folgenden Bestandteile :

2 g $NH_4NO_3$ (Stickstoffquelle), 1,4 g $Na_2HPO_4$, 0,6 g $KH_2PO_4$ (Puffer und Phosphorquelle), 0,2 g $MgSO_4 \cdot 7H_2O$, 0,01 g $CaCl_2 \cdot 2H_2O$, 0,001 g $FeSO_4 \cdot 7H_2O$ und 1 ml Spurenelementlösung (bestehend aus je 20 mg/l $Na_2MoO_4 \cdot 2H_2O$, $Na_2B_4O_7 \cdot 10H_2O$, $MnSO_4 \cdot H_2O$, $ZnSO_4 \cdot H_2O$ und $CuSO_4 \cdot 5H_2O$).

Man löst die Salze in destilliertem Wasser, bringt mit verdünnter Natronlauge auf pH 7 und füllt mit destilliertem Wasser auf 1 Liter auf. Zur Herstellung des festen Nährbodens MV 7-Agar werden der Nährlösung noch 20 g/l Agar (Difco) zugesetzt. Man sterilisiert im Autoklaven.

Die Inkubation erfolgt bei 28-30 °C. Einzelkolonien werden vorsichtig abgehoben und wiederum auf dem gleichen Medium ausgestrichen. Man wiederholt diese Prozedur mehrmals, bis reine Isolate vorliegen.

Isolierungsmethode 2

Eine Abwasserprobe oder eine Klärschlammsuspension (1 g pro 10 ml sterilem Wasser) wird in einen Schüttelkolben gegeben, worin sich sterile Nährlösung MV 7 befindet. Man gibt eine sterilfiltrierte wässrige Trimethyläthylammoniumchlorid-Lösung hinzu und inkubiert bei 28 °C als Standkultur 14 Tage oder als Schüttelkultur bei 250 Upm 7 Tage lang. 0,5 ml dieser ersten Anreicherungskultur werden in frische Nährlösung MV 7 gegeben und wieder bei 28 °C als Standkultur oder als Schüttelkultur inkubiert. 0,5 ml der zweiten Anreicherungskultur werden wieder in die frische Nährlösung gegeben und 7 Tage bei 28 °C inkubiert. Die zweite und dritte Anreicherungskultur wird auf sterilem Trimethylammoniumchloridhaltigem MV 7-Agar [Nährlösung MV 7 mit Zusatz von 20 g/l Agar (Difco)] plattiert und bei 28 °C inkubiert. Einzelkolonien werden vorsichtig abgehoben und wieder auf dem gleichen Medium ausgestrichen. Diese Prozedur wird mehrmals wiederholt, bis reine Isolate vorliegen.

Charakterisierung der neuen Mikroorganismen

1. Allgemeine Parameter und Mikroskopie

Alle in der Tabelle 1 aufgezählten Stämme sind gramnegativ, Oxidasepositiv und wachsen unter aeroben Bedingungen optimal bei 28° bis 30°.

Als C-Quellen können sie Alkylammoniumverbindungen der Formel

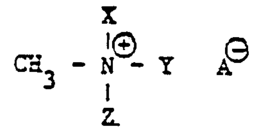

(IA)

worin A ein Anion, X Wasserstoff oder Methyl, Y und Z je Wasserstoff, Methyl oder Aethyl bedeuten, mit der Massgabe, dass X Wasserstoff bedeutet, wenn Y und Z Aethyl bedeuten, Alkylamin-N-oxide der Formel

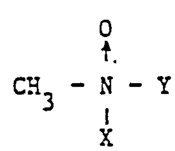

(IB)

worin X Wasserstoff oder Methyl und Y Wasserstoff, Methyl oder Aethyl bedeuten, sowie einige andere organische Verbindungen, z. B. Methanol (nur die Stämme Pseudomonas TMEA 14 und 199), Aethanol (nur die Stämme Pseudomonas TMEA 14, 81, 83, 84, 87, 89, 199 und 211) sowie Acetat, z. B. Natriumacetat und Glucose verwerten.

In der mikroskopischen Abbildung (Lichtmikroskop) sind sich alle Stämme sehr ähnlich und als motile Stäbchen von 1-2 μ Länge zu erkennen, welche einzeln, paarweise oder agglomeriert auftreten. Das elektronenmikroskopische Bild befindet sich in guter Uebereinstimmung mit den lichtmikroskopischen Beobachtungen. Alle Stämme sind sich auch dort sehr ähnlich, wobei vor allem zwei Zelltypen zu erkennen sind :

a) plumpe bis längliche Stäbchen mit 1-2 polaren bis subpolaren Geisseln und teilweise sehr feinen Haaren an der Zelloberfläche,

b) der andere Zelltypus hat die gleichen Merkmale wie a), besitzt aber keine Geisseln.

Elektronenmikroskopische Abbildung einiger typischer Zellen von Stämmen gemäss Tabelle 1

(Siehe Figuren Seite 6 f.)

2. Biochemische Charakterisierung und Klassifizierung der neuen Mikroorganismen

a) « Oxi/Ferm Tube »-Test (Roche)

Dieses Testsystem wird bei gramnegativen Stäbchen mit positiver Oxidasereaktion verwendet. Der Test wird parallel je zweimal mit Impfmaterial des betreffenden Stamms abgenommen von Trimethyläthylammoniumchlorid — Agar und Nutrient — Agar durchgeführt. Experimentelle Ausführung nach Vorschrift des Herstellers. Die Testresultate sind in Tabelle 2 zusammengestellt.

Zur allgemeinen biochemischen Charakterisierung und Klassifizierung der in Tabelle 1 aufgezählten Stämme werden zwei käufliche Test-systeme verwendet.

Tabelle 2

TMEA-Stämme getestet mit « Oxi/Ferm Tbe » (48 h, 28 °C)

| Kenn-Nr. | biochemischer Test | | | | | | | | |
|---|---|---|---|---|---|---|---|---|---|
| | anaerobe Dextrosespaltung | Arginin-dihydrolase | $N_2$-Produktion | $H_2S$-Bildung | Indol-Bildung | Xylosespaltung | aerobe Dextrosespaltung | Urease | Citratverwertung |
| TMEA 14 | − | − | −* | − | − | − | − | ± | − |
| TMEA 81 | − | −(*) | + | − | − | ± | ± | + | + |
| TMEA 83 | − | −(*) | ±* | − | − | ± | ± | ±(*) | + |
| TMEA 84 | − | − | −* | − | − | − | − | − | ±(*) |
| TMEA 86 | − | −* | −* | − | − | + | + | ± | −* |
| TMEA 87 | + | − | ±* | − | − | + | + | −(*) | ±* |
| TMEA 89 | − | −(*) | + | − | − | ± | ± | + | ±* |
| TMEA 199 | − | − | + | − | − | − | − | + | + |
| TMEA 211 | − | − | + | − | − | − | − | + | + |

+ beide Paralleltests positiv, — beide Paralleltests negativ, ± ein Test positiv, ein Test negativ, * im API 20E-Test positiv, (*) im API 20E-Test ±

Anhand der Testresultate in Tabelle 2 und nach deren numerischer Auswertung lassen sich die

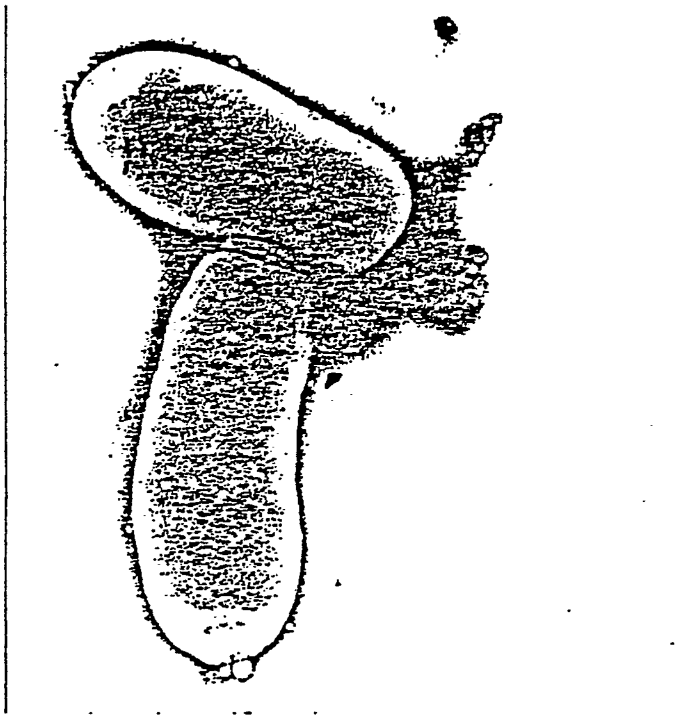

TMEA 14 32'500 x

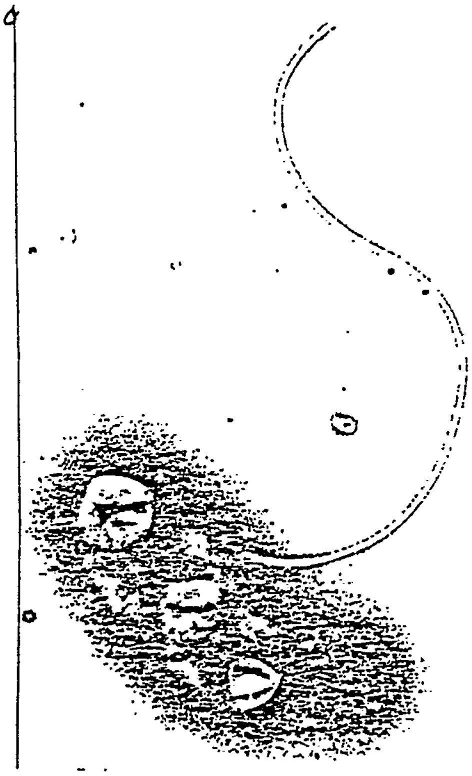

TMEA 81 32'500 x

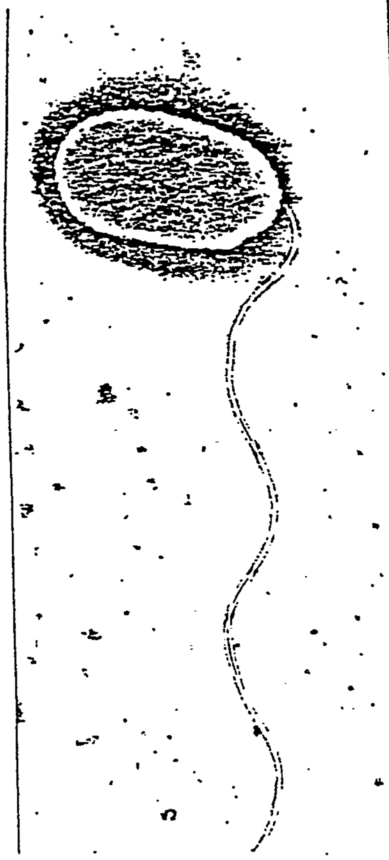

TMEA 211 22'1oo x

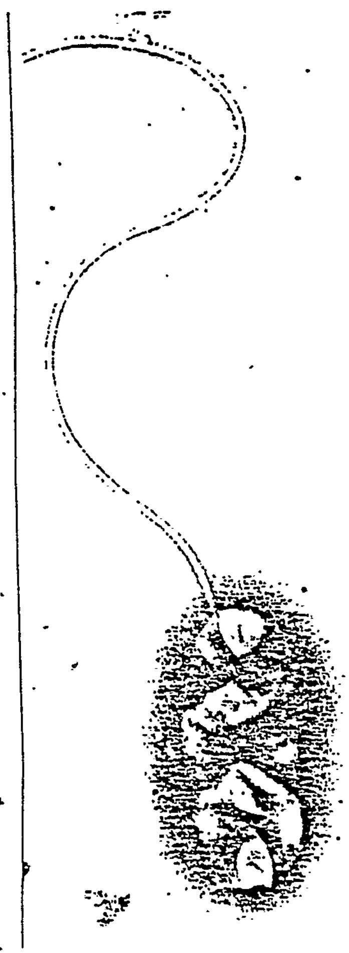

TMEA 89 32'500 x

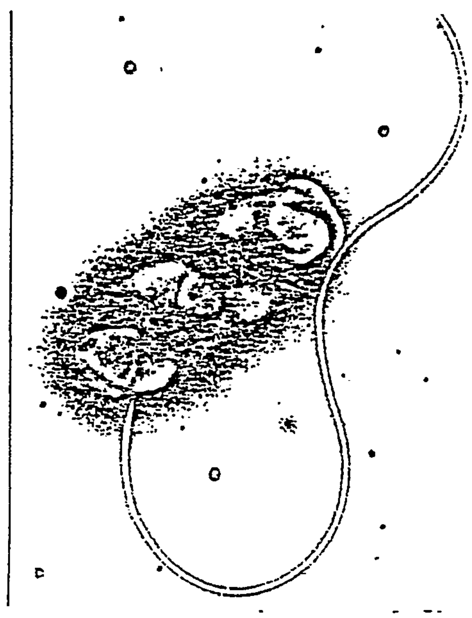

TMEA 87 32'500 x

einzelnen Stämme aufgrund ihres unterschiedlichen Grades an Uebereinstimmung zu vier Gruppen zusammenfassen und wie folgt nach numerischem Code annähernd zuordnen bzw. klassifizieren :

Tabelle 3

| Kenn.Nr. | Uebereinstimmung | Gruppe | Zuordnung |
|---|---|---|---|
| TMEA 14 und 84 | ähnlich | I | Pseudomonas ähnlich oder Pseudomonas Species |
| TMEA 86 und 87 | ähnlich | II | Pseudomonas ähnlich oder Pseudomonas Species |
| TMEA 81, 83 und 89 | sehr ähnlich | II | Achromobacter Species oder Pseudomonas Species |
| TMEA 199 und 211 | identisch | III | Achromobacter Species oder Alcaligenes faecalis |

b) API 20 E-Test

Der Test wird parallel je einmal mit Impfmaterial des betreffenden Stamms abgenommen von Trimethylammoniumchlorid-Agar und Nutrient-Agar durchgeführt. Experimentelle Ausführung nach Vorschrift des Herstellers. Die Testresultate sind in Tabelle 4 zusammengestellt.

(Siehe Tabelle 4 Seite 8 f.)

Nach den Testresultaten gemäss Tabelle 4 lassen sich die einzelnen Stämme in folgende Gruppen einordnen :

I' : TMEA 14 und 84 sind sich sehr ähnlich,
II' : TMEA 81, 83 und 89 sind sich sehr ähnlich,
III' : TMEA 199 und 211 sind praktisch identisch.
Die Stämme 86 und 87 sind wahrscheinlich Einzelstämme.

Die numerische Auswertung des API E-Tests erlaubt keine eindeutigen Zuordnungen, so dass die Resultate gemäss Tabelle 4 nur zur Charakterisierung der betreffenden Stämme herangezogen werden können.

c) Vergleich der beiden Testsysteme a) und b)

Es lassen sich für die neun Stämme gemäss Tabelle 1 folgende Quervergleiche anstellen :
$H_2S$-Bildung und Indol-Bildung sind in beiden Systemen immer negativ. Urease und Citratverwertung

Tabelle 4

TMEA-Stämme getestet mit API 20E (48 h, 28 °C)

| biochemischer Test | | TMEA 14 | TMEA 81 | TMEA 83 | TMEA 84 | TMEA 86 | TMEA 87 | TMEA 89 | TMEA 199 | TMEA 211 |
|---|---|---|---|---|---|---|---|---|---|---|
| CAT: | Katalase | + | + | + | + | + | + | + | + | + |
| $N_2$: | Nitratreduktion | + | + | + | + | + | + | + | + | + |
| $NO_2$: | Nitratreduktion | + | + | + | + | + | + | + | + | + |
| OX: | Oxidase | + s | + | + | + | + | + s | + | + | + |
| ARA: | L-Arabinoseverwertung | ± | — | — | — | — | + | — | — | — |
| AMY: | Amygdalinverwertung | + | — | — | + | — | + | ± | — | — |
| MEL: | Melibioseverwertung | — | — | — | — | + | + | — | — | — |
| SAC: | Sacharoseverwertung | ± | — | — | + | — | + | ± | — | — |
| RHA: | Rhamnoseverwertung | ± | — | — | — | — | + | — | — | — |
| SOR: | Sorbitverwertung | — | — | — | — | — | + | — | — | — |
| INO: | Inositverwertung | — | — | — | — | — | + | — | — | — |
| MAN: | Mannitverwertung | — | — | — | — | — | + | — | — | — |
| GLU: | Glucoseverwertung | — | — | — | ± | ± | + | — | — | — |
| GEL: | Gelatineverwertung | — | — | ± | — | — | + | — | — | — |
| VP: | Acetointest | ± | — | — | ± | ± | ± | ± | ± | ± |
| IND: | Tryptophanabbau Indolbildung | — | — | — | — | — | — | — | — | — |
| TDA: | Tryptophandesaminase | — | — | — | — | — | — | — | — | — |
| URE: | Urease | ± | ± | ± | — | (*) | ± | ± | + | + |
| $H_2S$: | Thiosulfatspaltung | — | — | — | — | — | — | — | — | — |
| CIT: | Citratverwertung | ± | + | ± | ± | + | + | + | + | + |
| ODC: | Ornithindecarboxylase | — | — | — | — | — | + | — | — | — |
| LDC: | Lysindercarboxylase | — | — | — | — | — | + | ± | — | — |
| ADH: | Arginindihydrolase | — | ± | ± | — | + | — | ± | — | — |
| ONPG: | Hydrolyse durch β-Galactosidase | ± | + | + | + | — | + | + | ± | — |

Kenn.-Nr.: TMEA 14, TMEA 81, TMEA 83, TMEA 84, TMEA 86, TMEA 87, TMEA 89, TMEA 199, TMEA 211

\+ beide Paralleltests positiv
— beide Paralleltests negativ
± ein Test positiv, ein Test negativ
(*) im Oxi/Ferm Tube ein Test positiv s = schwach
OX (Cytochromoxidase) bei $H_2S$ und ONPG
CAT : bei MAN, INO und SOR

stimmen in beiden Systemen relativ gut überein. Die unterschiedlichen Befunde hinsichtlich Arginindihydrolase und $N_2$-Produktion werden mit der höheren Empfindlichkeit der Bestimmungen im API 20 E-Test erklärt.

Die biochemischen Daten der neuen Mikroorganismen gemäss Tabelle 1, d. h. die Resultate der « Oxi/Ferm Tube »-Tests und « API-20-E » Tests, welche eine annähernde Klassifizierung der neuen Mikroorganismen als Pseudomonas species, Pseudomonas-ähnlich, Achromobacter species oder Alcaligenes faecalis erlauben, stimmen mit den in « Bergey's Manual Determinative Bacteriology » (8. Auflage) im Abschnitt über « Gram-negative Aerobic Rods and Cocci » für solche Mikroorganismen beschriebenen Merkmalen überein. Da sich Achromobacter species und Alcaligenes faecalis biochemisch praktisch nicht von Pseudomonas species unterscheiden und die Uebergänge zwischen den Genera fliessend sind, wird aufgrund des charakteristischen Merkmals der polaren bis subpolaren Begeisselung die Bezeichnung « Pseudomonas » für alle Mikroorganismen der vorliegenden Erfindung gewählt.

Konservierung der Stämme

Für die Konservierung der Stämme gemäss Tabelle 1 eignen sich folgende Methoden :

a) Adsorption des Zellmaterials des betreffenden Stamms auf Glaskügelchen in Glycerinlösung und anschliessender Lagerung bei — 20 °C,

b) Aufbewahrung des Zellmaterials des betreffenden Stamms über Schrägagar und

c) Lyo-Ampullen. Die betreffende Kultur wird von der Nährlösung abzentrifugiert und die Zellmasse in 1/4 bis 1/3-Volumen 15 %iger Ski Milk resuspendiert und lyophilisiert.

Von den in der Tabelle 1 aufgezählten Stämmen können sich spontan Mutanten bilden, oder es lassen sich künstlich Mutanten herstellen, die ebenso wie die natürlichen Stämme. Trimethyläthylammoniumsalze, in wässriger Lösung abbauen können und Zellmasse produzieren. Solche Mutanten kann man chemisch, z. B. mit gewissen Guanidinderivaten, z. B. N-Methyl-N'-nitro-N-nitrosoguanidin oder Alkalinitrit, z. B. Natriumnitrit, oder physikalisch, z. B. durch Ultraviolett-, Röntgen- oder radioaktive Strahlung, erzeugen.

Die erfindungsgemässen Mikroorganismen finden Anwendung in einem Verfahren zur mikrobiologischen Reinigung von wässrigen Lösungen, welches dadurch gekennzeichnet ist, dass man in einer wässrigen Lösung, welche Trimethyläthylammoniumsalze enthalten, einen Mikroorganismus des Genus Pseudomonas aus der obengenannten Gruppe welcher die Zellmasse mit einem Vielfachen der ungefähren Summenformel $C_5H_9NO_2$ und einem Gehalt von 44,67 % C, 7,04 % H, 10,50 % N, 25,00 % O, 1,12 % P und 0,33 % S, sowie ca. 8 % $H_2O$ (lyophilisiert) zu produzieren vermag, sowie eine von diesem Mikroorganismus abgeleitete, für das Verfahren verwendbare Mutante, welche ebenfalls diese Zellmasse produziert, in Gegenwart von esentiellen anorganischen Salzen und gegebenenfalls einer Stickstoffquelle bei ca. 20 °C bis ca. 40 °C und einem pH-Wert von ca. 4 bis ca. 7,5 züchtet und, wenn erwünscht, die erhältliche Zellmasse isoliert.

Bei der Züchtung oder Fermentation bauen die in Tabelle 1 aufgezählten Stämme die in wässriger Lösung, z. B. in einem Abwasser, befindlichen Trimethyläthylammoniumsalze ab und verbrauchen Sauerstoff. Diese Bestandteile können z. T. in sehr hohen Konzentrationen der betreffenden Verbindungen oder Salze, von den in Tabelle 1 genannten Stämmen abgebaut werden. Beim Abbauverfahren produzieren die Mikroorganismen Zellmasse, gekennzeichnet durch ein Vielfaches der ungefähren Summenformel $C_5H_9NO_2$ und einen Gehalt von 44,67 % C, 7,04 % H, 10,50 % N, 25,00 % O, 1,12 % P und 0,33 % S, sowie ca. 8 % $H_2O$ (lyophilisiert). Als weitere Fermentationsprodukte werden Kohlendioxid, Ammoniumsalze der Formel $NH_4^{\oplus}A^{\ominus}$, z. B. Ammoniumchlorid, und die Säure HA, z. B. Chlorwasserstoff, gebildet.

Der pH-Wert ist durch Zugabe von Pufferlösung, z. B. Phosphatpufferlösung, oder von wässriger Base, z. B. wässriger Natrium- oder Kaliumhydroxidlösung auf Werte zwischen ca. 4,0 und 7,5, bevorzugt ca. 5 bis 6, einzustellen.

Die Züchtung erfolgt in Gegenwart von essentiellen anorganischen Salzen. Solche Salze sind beispielsweise Halogenide, z. B. Chloride, Carbonate, Sulfate oder Phosphate von Alkali-, Erdalkali- oder Uebergangsmetallen, sowie Borate oder Molybdate von Alkalimetallen.

Bevorzugte essentielle anorganische Salze sind beispielsweise Dinatrium- oder Dikaliumhydrogenphosphat, Natrium- oder Kaliumdihydrogenphosphat, Magnesium- und Eisensulfat und Kalium- und Calciumchlorid. In geringen Mengen können noch Zink-, Mangan- und Kupfersulfat, Natriummolybdat und Borax zugesetzt werden.

Die Züchtung erfolgt unter aeroben Bedingungen, z. B. unter Sauerstoff- oder Luftzufuhr, und zwar unter Schütteln oder Rühren in Schüttelkolben oder Fermentern. Die Züchtung lässt sich in einem Temperaturbereich von ca. 25° bis ca. 35 °C, bevorzugt bei ca. 27° bis ca. 28 °C, durchführen.

Die Züchtung kann ansatzweise, z. B. durch ein- oder mehrmalige Zugabe von Nährlösung, oder kontinuierlich durch dauernde Zugabe von Nährlösung erfolgen.

Vorzugsweise züchtet man in mehreren Stufen, indem man zunächst eine oder mehrere Vorkulturen, z. B. in einem flüssigen Nährmedium, herstellt, welche man anschliessend in die eigentliche Hauptkultur

überimpft. Eine Vorkultur lässt sich beispielsweise herstellen, indem man eine Probe mit Zellmaterial des betreffenden Mikroorganismus, der beispielsweise über Schrägagar aufbewahrt wird, in eine sterile Nährlösung, z. B. MV 7, mit einer geeigneten Kohlenstoffquelle, z. B. Trimethyläthylammoniumchlorid, überträgt und mehrere Tage bei 28° inkubiert. Mit dieser ersten Vorkultur impft man frische Nährlösung, z. B. MV 7, mit derselben Kohlenstoffquelle an und inkubiert nochmals mehrere Tage bei 28°.

Man kann den Fermentationsverlauf durch Probenentnahme analytisch während der Fermentation verfolgen, z. B. durch Messung des pH-Wertes der Kultur oder der optischen Dichte, welche ein Mass für das Wachstum des jeweiligen Stamms ist, sowie gravimetrisch anhand des Trockengewichts der gebildeten Zellmasse.

Die gebildete Zellmasse lässt sich z. B. anschliessend nach einem der zahlreichen in der Europäischen Patentschrift 0 010 243 beschriebenen Verfahren aufarbeiten und z. B. in Düngemittel überführen.

Als Zellmasse sind im vorliegenden Fall alle sich in lebendem Zustand, z. B. im Teilungszustand bzw. Ruhezustand, im partiellen oder vollständigen Zelltod, oder bereits in der enzymatischen Zersetzung oder in der Zersetzung durch Fremdkulturen befindlichen Zellsysteme zu verstehen, welche von den Mikroorganismen der vorliegenden Anmeldung aufgebaut werden.

Diese Zellmasse ist ein wertvoller Rohstoff, der eine definierte und reproduzierbare Zusammensetzung hat. Ungefähre Summenformel : $C_5H_9NO_2$, Gehalt (Lyophilisat) : 44,67 % C, 7,04 % H, 10,50 % N, 25,00 % O, 1,12 % P und 0,33 % S, sowie ca. 8 % $H_2O$.

Die verfahrensgemäss erhältliche Zellmasse kann als Einzellerprotein mit definierter und reproduzierbarer Zusammensetzung beispielsweise als Viehfutterzusatz verwendet werden. Die Zellmasse lässt sich auch beispielsweise als Suspension oder aufgearbeitet, z. B. nach Entwässerung oder Pasteurisierung, als Düngemittel verwenden. Man kann die Zellmasse auch als Ausgangsmaterial zur Herstellung von Biogas mit hohem Heizwert (Zusammensetzung ca. 70 % Methan, 29 % Kohlendioxid und 1 % Wasserstoff, Heizwert ca. 5 500-6 500 kcal/$m^3$), beispielsweise durch anaerobe Vergärung in Faultürmen, verwenden. Der Rückstand (Faulschlamm) aus dem Herstellungsverfahren von Biogas ist ebenfalls ein hochwertiger Dünger, der im Vergleich zur ursprünglichen Zellmasse mit Stickstoff stark angereichert ist.

Die folgenden Beispiele illustrieren die vorliegende Erfindung. Die Temperaturangaben erfolgen in Grad Celsius.

### Beispiel 1 (Herstellung der Vorkultur)

1 Probe mit auf Schrägagar aufbewahrtem Zellmaterial des Mikroorganismus vom Stamm TMEA 199 wird in einen Schüttelkolben, enthaltend 20 ml Nährlösung MV 7 mit der weiter vorn angegebenen Zusammensetzung und 5 g/l Trimethyläthylammoniumchlorid gegeben und 72 Stunden bei 28° und 250 Upm inkubiert. 5-7 ml dieser ersten Vorkultur werden in einen zweiten Schüttelkolben, enthaltend 100 ml Nährlösung MV 7 (ohne Ammoniumnitrat) und 5 g/l Trimethyläthylammoniumchlorid und 5 mMol Phosphatpuffer (pH 7) gegeben und 72 Stunden bei 28° und 250 Upm inkubiert.

### Beispiel 2

Analog Beispiel 1 lassen sich Vorkulturen der Stämme TMEA 14, 81, 83, 84, 86, 87, 89 oder 211 herstellen.

### Beispiel 3

In einem Laborfermenter werden 10 l gegebenenfalls hitzesterilisierte (Sterilisation 20 Min. bei 120°) Nährlösung MV 7 (ohne Ammoniumnitrat) mit einer gegebenenfalls sterilfiltrierten Trimethyläthylammoniumchlorid-Lösung so vereinigt, dass ein ungefähres Arbeitsvolumen von 10 l mit einer Konzentration von 10 g/l Trimethyläthylammoniumchlorid resultiert.

Man gibt eine Probe mit ca. 500 ml der zweiten Vorkultur des Stamms TMEA 199 hinzu und hält folgende Bedingungen ein : pH-Wert von 5,5, welcher durch Zugabe jeweils von 4-normaler Natronlauge und 1-normaler Salzsäure konstant gehalten wird, Temperatur : 28°, Luftzufuhr 0,26 l/l min und Rührgeschwindigkeit von 400-700 Upm.

Der Stamm wächst auf reinem Trimethyläthylammoniumchlorid als einziger Kohlenstoff- und Stickstoffquelle. Nach ca. 200 Stunden ist das eingesetzte Trimethyläthylammoniumchlorid vollständig abgebaut. Die gebildete Zellmasse liegt als Gemisch von Einzelzellen oder Aggregaten verschiedener Grösse vor, welche man durch Absetzen oder Zentrifugieren abtrennen kann.

### Beispiel 4

Analog Beispiel 3 lässt sich Trimethyläthylammoniumchlorid in wässriger Lösung abbauen, indem man in einem Laborfermenter Vorkulturen der Stämme TMEA 14, 81, 83, 84, 86, 87, 89 oder 211 züchtet.

### Beispiel 5

In einem Laborfermenter werden 10 l hitzesterilisierte (Sterilisation 20 Min. bei 120°) Nährlösung MV

7 (ohne Ammoniumnitrat) mit einer sterilfiltrierten Trimethyläthylammoniumchlorid-haltigen Abwasserlösung (Zusammensetzung : 45,2 % Trimethyläthylammoniumchlorid, 6,4 % HCl, 47,5 % Wasser und weniger als 1 % Aromaten) vereinigt, so dass ein ungefähres Arbeitsvolumen von 10 l mit einer Konzentration von ca. 10 g/l Trimethyläthylammoniumchlorid resultiert. Man gibt eine Probe mit ca. 500 ml der zweiten Vorkultur des Stamms TMEA 199 hinzu und hält die im Beispiel 3 genannten Bedingungen ein. Ein vollständiger Abbau des Substrats erfolgt innerhalb von 190 Stunden.

Beispiel 6

Analog Beispiel 5 lässt sich Trimethyläthylammoniumchlorid in sterilfiltrierten Abwasserlösungen abbauen, indem man in einem Laborfermenter Vorkulturen der Stämme TMEA 14, 81, 83, 84, 86, 87, 89 oder 211 züchtet.

Beispiel 7

Analog Beispiel 5 und 6 lässt sich unter Verzicht auf Sterilfiltration Trimethyläthylammoniumchlorid in Abwasserlösungen abbauen, indem man in einem Laborfermenter Vorkulturen der Stämme TMEA 14, 81, 83, 84, 86, 87, 89, 199 oder 211 züchtet.

**Patentansprüche**

1. Mikroorganismen des Genus Pseudomonas aus der Gruppe der folgenden Stämme : Pseudomonas TMEA 14 (NRRL-B-12582), TMEA 81 (NRRL-B-12581), TMEA 83 (NRRL-B-12580), TMEA 84 (NRRL-B-12579), TMEA 86 (NRRL-B-12578), TMEA 87 (NRRL-B-12577), TMEA 89 (NRRL-B-12576), TMEA 199 (NRRL-B-12575) und TMEA 211 (NRRL-B-12574) oder eine davon abgeleitete Mutante, welche in wässriger Lösung Trimethyläthylammoniumsalze abbauen und dabei Zellmasse produzieren kann.

2. Mikroorganismen des Genus Pseudomonas gemäss Anspruch 1 aus der Gruppe der folgenden Stämme : Pseudomonas TMEA 14 (NRRL-B-12582), TMEA 81 (NRRL-B-12581), TMEA 83 (NRRL-B-12580), TMEA 84 (NRRL-B-12579), TMEA 86 (NRRL-B-12578), TMEA 87 (NRRL-B-12577), TMEA 89 (NRRL-B-12576), TMEA 199 (NRRL-B-12575) und TMEA 211 (NRRL-B-12574).

3. Verfahren zur mikrobiologischen Reinigung von wässrigen Lösungen enthaltend Trimethyläthylammoniumsalze, dadurch gekennzeichnet, dass man in einer Trimethyläthylammoniumsalze-haltigen wässrigen Lösung einen Mikroorganismus des Genus Pseudomonas aus der Gruppe der Stämme : Pseudomonas TMEA 14 (NRRL-B-125582), TMEA 81 (NRRL-B-12581), TMEA 83 (NRRL-B-12580), TMEA 84 (NRRL-B-12579), TMEA 86 (NRRL-B-12578), TMEA 87 (NRRL-B-12577), TMEA 89 (NRRL-B-12576), TMEA 199 (NRRL-B-12575) und TMEA 211 (NRRL-B-12574) oder eine davon abgeleitete, für das Verfahren geeignete Mutante gemäss Anspruch 1 in Gegenwart von essenziellen anorganischen Salzen und gegebenenfalls einer Stickstoffquelle bei ca. 20 °C bis ca. 40 °C und einem pH-Wert von ca. 4 bis ca. 7,5 züchtet und, wenn erwünscht, die erhältliche Zellmasse isoliert.

4. Verfahren nach Anspruch 3 zur mikrobiologischen Reinigung von wässrigen Lösungen enthaltend Trimethyläthylammoniumchlorid, dadurch gekennzeichnet, dass man in einer Trimethyläthylammoniumchlorid-haltigen wässrigen Lösung einen Mikroorganismus des Genus Pseudomonas aus der Gruppe der folgenden Stämme : Pseudomonas TMEA (NRRL-B-12582), TMEA 81 (NRRL-B-12581), TMEA 83 (NRRL-B-12580), TMEA 84 (NRRL-B-12579), TMEA 86 (NRRL-B-12578), TMEA 87 (NRRL-B-12577), TMEA 89 (NRRL-B-12576), TMEA 199 (NRRL-B-12575) und TMEA 211 (NRRL-B-12574) züchtet.

5. Verfahren nach Anspruch 3 oder 4, dadurch gekennzeichnet, dass man die Züchtung bei 28 °C durchführt.

6. Verfahren nach einem der Ansprüche 3-5, dadurch gekennzeichnet, dass man die Züchtung kontinuierlich durchführt.

**Claims**

1. Microorganisms of the genus Pseudomonas selected from the group of the following strains : Pseudomonas TMEA 14 (NRRL-B-12582), TMEA 81 (NRRL-B-12581), TMEA 83 (NRRL-B-12580), TMEA 84 (NRRL-B-12579), TMEA 86 (NRRL-B-12578), TMEA 87 (NRRL-B-12577), TMEA 89 (NRRL-B-12576), TMEA 199 (NRRL-B-12575) and TMEA 211 (NRRL-B-12574) or a mutant derived therefrom which, in aqueous solution, is able to degrade trimethylethylammonium salts, thereby producing biomass.

2. Microorganisms of the genus Pseudomonas according to claim 1, selected from the group of the following strains : Pseudomonas TMEA 14 (NRRL-B-12582), TMEA 81 (NRRL-B-12581), TMEA 83 (NRRL-B-12580), TMEA 84 (NRRL-B-12579), TMEA 86 (NRRL-B-12578), TMEA 87 (NRRL-B-12577), TMEA 89 (NRRL-B-12576), TMEA 199 (NRRL-B-12575) and TMEA 211 (NRRL-B-12574).

3. A process for the microbiological purification of aqueous solutions containing trimethylammonium salts, which process comprises culturing in an aqueous solution containing trimethylethylammonium salts a microorganism of the genus Pseudomonas selected from the group of the following strains : Pseudomonas TMEA 14 (NRRL-B-12582), TMEA 81 (NRRL-B-12581), TMEA 83 (NRRL-B-12580), TMEA 84 (NRRL-B-12579), TMEA 86 (NRRL-B-12578), TMEA 87 (NRRL-B-12577), TMEA 89 (NRRL-B-12576), TMEA 199 (NRRL-B-12575) and TMEA 211 (NRRL-B-12574) or a mutant, according to claim 1, which is derived therefrom and is suitable for the process, in the presence of nutrient inorganic salts and optionally of a nitrogen source, in the temperature range from about 20 °C to about 40 °C and in a pH range from about 4 to about 7.5, and, if desired, isolating the resultant biomass.

4. A process according to claim 3 for the microbiological purification of aqueous solutions containing trimethylethylammonium chloride, which process comprises culturing in an aqueous solution containing trimethylethylammonium chloride a microorganism of the genus Pseudomonas selected from the group of the following strains : Pseudomonas TMEA 14 (NRRL-B-12582), TMEA 81 (NRRL-B-12581), TMEA 83 (NRRL-B-12580), TMEA 84 (NRRL-B-12579), TMEA 86 (NRRL-B-12578), TMEA 87 (NRRL-B-12577), TMEA 89 (NRRL-B-12576), TMEA 199 (NRRL-B-12575) and TMEA 211 (NRRL-B-12574).

5. A process according to either of claims 3 or 4, wherein culturing is carried out at 28 °C.

6. A process according to any one of claims 3 to 5, wherein culturing is carried out continuously.

**Revendications**

1. Microorganismes du genre pseudomonas, du groupe des souches suivantes : Pseudomonas TMEA 14 (NRRL-B-12582), TMEA 81 (NRRL-B-12581), TMEA 83 (NRRL-B-12580), TMEA 84 (NRRL-B-12579), TMEA 86 (NRRL-B-12578), TMEA 87 (NRRL-B-12577), TMEA 89 (NRRL-B-12576), TMEA 199 (NRRL-B-12575) et TMEA 211 (NRRL-B-12574) ou un mutant dérivant de ces souches, capables de dégrader les sels de triméthyléthylammonium en solution aqueuse et de produire simultanément une masse cellulaire.

2. Microorganismes du genre pseudomonas selon la revendication 1, du groupe des souches suivantes : Pseudomonas TMEA 14 (NRRL-B-12582), TMEA 81 (NRRL-B-12581), TMEA 83 (NRRL-B-12580), TMEA 84 (NRRL-B-12579), TMEA 86 (NRRL-B-12578), TMEA 87 (NRRL-B-12577), TMEA 89 (NRRL-B-12576), TMEA 199 (NRRL-B-12575) et TMEA 211 (NRRL-B-12574).

3. Procédé pour la purification microbiologique de solutions aqueuses contenant des sels de triméthyléthylammonium, caractérisé en ce que l'on cultive dans une solution aqueuse contenant des sels de triméthyléthylammonium un microorganisme du genre pseudomonas, du groupe des souches : Pseudomonas TMEA 14 (NRRL-B-12582), TMEA 81 (NRRL-B-12581), TMEA 83 (NRRL-B-12580), TMEA 84 (NRRL-B-12579), TMEA 86 (NRRL-B-12578), TMEA 87 (NRRL-B-12577), TMEA 89 (NRRL-B-12576), TMEA 199 (NRRL-B-12575) et TMEA 211 (NRRL-B-12574) ou un mutant dérivant de ces souches, approprié à l'opération, selon la revendication 1, en présence de sels minéraux essentiels et le cas échéant d'une source d'azote, à une température d'environ 20 à 40 °C et un pH d'environ 4 à 7,5 et, si on le désire, on isole la masse cellulaire obtenue.

4. Procédé selon la revendication 3, pour la purification microbiologique de solutions aqueuses contenant du chlorure de triméthyléthylammonium, caractérisé en ce que l'on cultive dans une solution aqueuse contenant du chlorure de triméthyléthylammonium un microorganisme du genre pseudomonas, du groupe des souches suivantes : Pseudomonas TMEA 14 (NRRL-B-12582), TMEA 81 (NRRL-B-12581), TMEA 83 (NRRL-B-12580), TMEA 84 (NRRL-B-12579), TMEA 86 (NRRL-B-12578), TMEA 87 (NRRL-B-12577), TMEA 89 (NRRL-B-12576), TMEA 199 (NRRL-B-12575) et TMEA 211 (NRRL-B-12574).

5. Procédé selon la revendication 3 ou 4, caractérisé en ce que la culture est réalisée à 28 °C.

6. Procédé selon l'une des revendications 3 à 5, caractérisé en ce que la culture est réalisée en continu.